# EUROPEAN PATENT APPLICATION

(11) **EP 4 541 312 A1**
(43) Date of publication of application: **23.04.2025**
(21) Application number: 23842391.7
(22) Date of filing: 20.07.2023
(51) Int. Cl.: A61F 2/07, A61F 2/90

(54) **STENT IMPLANT AND PREPARATION METHOD THEREFOR**

(30) Priority: 22.07.2022 CN 202210868780
(71) Applicant: Shanghai Intervascular MedTech Co., Ltd., Shanghai 201318 (CN)
(72) Inventor: ZHANG, Yu, Shanghai 201318 (CN); CAO, Yang, Shanghai 201318 (CN); WANG, Jinyao, Shanghai 201318 (CN); ZHU, Qing, Shanghai 201318 (CN)
(74) Representative: Marks & Clerk LLP
(86) International application number: PCT/CN2023/108374
(87) International publication number: WO 2024/017329

(57) **Abstract**

A stent implant and a method of preparing the same are provided. The stent implant includes a stent-graft (110) and a braided stent (120). At least one axial end of the stent-graft (110) is connected to the braided stent (120). The stent-graft (110) includes a cutting stent (111) and a coating (112). Metal coverage of the cutting stent (111) is 10% to 20%. Surfaces of stent struts in the cutting stent (111) are coated with a connecting layer for enhancing adhesion force of the coating (112) to the stent struts. The coating (112) includes two first membranes (112a): an outer first membrane (112a) disposed over an outer surface of the cutting stent (111); and an inner first membrane (112a) disposed over an inner surface of the cutting stent (111). The outer first membrane (112a) has lower air permeability than the inner first membrane (112a). With this arrangement, the stent implant provides both increased radial support and desirable compliance and is associated with a lower risk of a lumen of the stent-graft (110) becoming stenotic or occluded.

## Description

### TECHNICAL FIELD

The present invention pertains to the field of medical devices and, in particular, relates to a stent implant and a method of preparing the same.

### BACKGROUND

Persistently elevated portal pressure caused by liver cirrhosis, obstruction of the portal vein trunk or hepatic vein, or other unknown reasons is commonly known as portal hypertension. Its hematological manifestations include: portal blood fails to pass through the liver to flow back to the inferior vena cava, elevating portal pressure; and a traffic branch between the portal and systemic veins opens, and a large proportion of portal blood enters the systemic circulation through the traffic branch without passing through the liver. Its clinical symptoms include enlarged thoracoepigastric and esophageal veins, splenomegaly and hypersplenism, hepatic functional decompensation and ascites, portal hypertensive gastrointestinal vascular disease and so forth. In severe cases, gastroesophageal variceal hemorrhage may develop.

Transjugular intrahepatic portosystemic shunt (TIPS) is developed on the basis of the increasingly established transjugular portographic and percutaneous Transjugular Hepatic Puncture techniques. In clinical applications, a TIPS procedure may involve implanting a stent-graft into liver tissue. However, the postoperative healing and growth of the liver tissue will try to repair the shunt created in the procedure, exerting compression on the stent-graft. Non-metallic regions of the stent-graft, where there is no metal support, would experience faster liver tissue growth toward the lumen of the stent-graft, and the luminal surface may therefore become uneven, reducing the effective cross-sectional area of the lumen for blood flow and slowing down blood flow within the lumen of the stent-graft. Consequently, thrombosis may occur, which will additionally narrow or even occlude the lumen of the stent. Further, due to poor compliance, currently available stent-grafts for use in TIPS procedures cannot adapt themselves to the geometry of a blood vessel or a shunt created in tissue, but tend to straighten the blood vessel or shunt. In particular, when used in a blood vessel or at a tissue site with large curvature, such a stent-graft tends to become "capped" at one end, impeding blood flow therethrough and possibly causing damage to the blood vessel.

### SUMMARY OF THE INVENTION

It is an object of the present invention to provide a stent implant providing both enhanced support and desirable compliance and associated with a lower risk of a lumen of the stent implant becoming stenotic or occluded and lower possibility of causing damage to a blood vessel, and a method of preparing such a stent implant.

To this end, the present invention provides a stent implant comprising a stent-graft and a braided stent. A least one axial end of the stent-graft is connected to the braided stent. The stent-graft comprises a cutting stent and a coating. Metal coverage of the cutting stent is about 10% to 20%. Surfaces of stent struts in the cutting stent are coated with a connecting layer for enhancing adhesion force of the coating to the stent struts. The coating comprises two first membranes: an outer first membrane disposed over an outer surface of the cutting stent; and an inner first membrane disposed over an inner surface of the cutting stent. The outer first membrane has lower air permeability than the inner first membrane.

In one embodiment, the coating further comprises at least one second membrane having a lower glass transition temperature than the first membrane, wherein the second membrane is disposed between the outer and inner first membranes at a proximal end of the cutting stent, and/or the second membrane is disposed between the outer and inner first membranes at a distal end of the cutting stent, and wherein the second membrane is configured to enhance an adhesion force between the outer and inner first membranes.

In one embodiment, the first membrane is made of an expanded polytetrafluoroethylene (ePTFE) material, and the second membrane is made of a fluorinated ethylene propylene (FEP) material.

In one embodiment, a width of the second membrane disposed at the proximal or distal end of the cutting stent along an axis of the stent-graft accounts for 3% to 18% of a total axial length of the stent-graft.

In one embodiment, an air permeability time of the outer first membrane is 6.0 s to 20.0 s, an air permeability time of the inner first membrane is 0.5 s to 6.0 s, the first membrane has a thickness of 20 µm to 180 µm, and the second membrane has a thickness of 10 µm to 30 µm.

In one embodiment, the coating has an extension part extending beyond one of the axial ends of the cutting stent, wherein an extension part of the second membrane is disposed between an extension part of the outer first membrane and an extension part of the inner first membrane, and wherein the extension part of the second membrane covers a surface of a portion of the braided stent proximate a corresponding end of the cutting stent.

In one embodiment, the coating is thermally fused to the braided stent, and/or the stent implant further comprises a plurality of connecting bands, each passed through a corresponding mesh opening in the braided stent at an interface of the braided stent and the cutting stent and thermally fused to the coating.

In one embodiment, the connecting layer is made of an FEP or ePTFE material.

In one embodiment, the connecting layer is formed on the surfaces of the stent struts by dipping the entire cutting stent in an ePTFE or FEP solution, or by spraying an ePTFE or FEP solution on the entire cutting stent.

In one embodiment, the braided stent is braided with at least one wire, wherein the braided stent comprises first structural regions and second structural regions, wherein wire portions overlap in the first structural region, wherein wire portions are in mutual engagement in the second structural region, and wherein the first structural regions are arranged into a helical pattern along an axis of the braided stent.

In one embodiment, the stent struts include transverse stent struts and longitudinal stent struts, wherein the transverse stent struts forms a plurality of annular stent sections arranged side by side along an axis of the cutting stent, wherein the longitudinal stent struts connect the annular stent sections, wherein any adjacent annular stent sections are connected by a plurality of longitudinal stent struts, and wherein only some crests or troughs in each annular stent section are connected to the longitudinal stent struts.

To the above end, the present invention also provides a method of preparing the stent implant as defined in any of the preceding paragraphs. The method comprises:
disposing the inner first membrane over a mold;
sequentially disposing the cutting stent and the braided stent over the inner first membrane so that the entire inner surface of the cutting stent and an inner surface of a portion of the braided stent proximate one end of the cutting stent is covered by the inner first membrane;
disposing the outer first membrane over the cutting stent, wherein the outer first membrane covers the entire outer surface of the cutting stent and an outer surface of the braided stent proximate the one end of the cutting stent; and
covering the outer first membrane by a heat shrink tube, then performing a heating and cooling cycle and finally removing the mold and the heat shrink tube.

In one embodiment, after the inner first membrane is disposed over the mold and before the cutting stent and the braided stent are disposed over the inner first membrane, the second membrane having a lower glass transition temperature than the first membrane is disposed between the inner first membrane and the inner surface of the cutting stent at at least one axial end of the cutting stent to enhance adhesion force between the outer and inner first membranes.

Additionally, after the cutting stent and the braided stent are disposed, a distal radiopaque marker band is wrapped around an interface of the cutting stent and the braided stent. Further, the outer first membrane is disposed over the cutting stent after the disposal of the distal radiopaque marker band.

In one embodiment, the method further comprises, after the cutting stent and the braided stent are disposed over the inner first membrane and before the outer first membrane is disposed over the cutting stent:
disposing the second membrane having a lower glass transition temperature than the first membrane between the outer surface of the cutting stent and the outer first membrane at at least one axial end of the cutting stent to enhance adhesion force between the outer and inner first membranes; and
after the disposal of the second membrane, wrapping a distal radiopaque marker band around an interface of the cutting stent and the braided stent, wherein the outer first membrane is disposed over the cutting stent after the disposal of the distal radiopaque marker band.

In one embodiment, the method further comprises, before the outer first membrane is disposed over the cutting stent: disposing the plurality of connecting bands at one axial end of the cutting stent, wherein each connecting bands is passed through a corresponding mesh opening in the braided stent at the interface of the braided stent and the cutting stent, thereby engaging some mesh openings of the braided stent that are proximate said one end of the cutting stent.

In one embodiment, the method further comprises: wrapping the heat shrink tube with a film; and then performing a thermal treatment process.

The present invention provides a stent implant and a method of preparing the same. The stent implant includes a stent-graft and a braided stent. A least one axial end of the stent-graft is connected to the braided stent. The stent-graft comprises a cutting stent and a coating. Metal coverage of the cutting stent is about 10% to 20%. Surfaces of stent struts in the cutting stent are coated with a connecting layer for enhancing adhesion force of the coating to the stent struts. The coating comprises two first membranes: an outer first membrane disposed over an outer surface of the cutting stent; and an inner first membrane disposed over an inner surface of the cutting stent. The outer first membrane has lower air permeability than the inner first membrane.

With this arrangement, the stent implant of the present invention provides both good support and desirable compliance. For example, when used in a transjugular intrahepatic portosystemic shunt (TIPS) procedure, the less air-permeable outer first membrane disallows the permeation of bile due to a denser texture, and the two first membranes with different degrees of air permeability impart increased compliance to the stent-graft. In this way, the influence of the thermal fusing processes on the compliance of the stent-graft is mitigated, and in addition to providing better support and maintaining a blood flow shunt, the stent-graft can adapt itself to the geometry of the blood flow shunt and reduce damage to a blood vessel. Additionally, the outer and inner first membranes with different degrees of air permeability can be fused and joined to each other with higher strength and no delamination during use, reducing the risk of a lumen of the stent-graft becoming stenotic or occluded. For example, during thermal fusion of the coating, the different pore sizes of the two first membranes allow the molten material of each membrane to desirably more penetrate into the other membrane, with increased penetration, a stronger adhesion force between the two first membranes can be achieved. Further, the metal coverage of the cutting stent is as high as 10% to 20%, which enables the stent-graft to provide both increased radial support and desirable compliance. Furthermore, the connecting layer that coats the surface of the stent struts enhances adhesion force of the coating to the stent struts and reduces the risk of the coating being separated from the stent struts, thereby further lowering the risk of the lumen of the stent-graft becoming stenotic or occluded.

### BRIEF DESCRIPTION OF THE DRAWINGS

In order to more clearly explain embodiments of the present invention or the prior art, the accompanying drawings, to which reference is to be made in connection with the following description of the embodiments, will be briefed below. Apparently, these drawings show only some embodiments of the present invention, and those of ordinary skill in the art can obtain other drawings in light of those contained herein, without paying any creative effort.
Fig. 1 shows an overview of a stent implant according to a preferred embodiment of the present invention, in which a braided stent is connected to a stent-graft by connecting bands passed through the most proximal mesh openings in braided stent and thermally fused to a coating.
Fig. 2 shows an overview of a stent implant according to a preferred embodiment of the present invention, in which a braided stent is directly thermally fused to a coating in a stent-graft.
Fig. 3 shows a partial view of a cutting stent according to a preferred embodiment of the present invention.
Fig. 4 is an unrolled plan view of a braided stent according to a preferred embodiment of the present invention, showing first and second structural regions thereof.
Fig. 5 is an unrolled plan view of a braided stent according to a preferred embodiment of the present invention, showing first structural regions thereof arranged into a helical pattern.
Fig. 6 is a partial view showing a connecting band disposed at an interface of a braided stent and a stent-graft according to a preferred embodiment of the present invention.
Fig. 7 shows bulging of a coating toward the interior of a stent-graft as a consequence of being squeezed by liver tissue in a comparative example.
Fig. 8 schematically illustrates an end face of a stent implant according to a preferred embodiment of the present invention.

### List of Reference Numerals

110-stent-graft; 111-cutting stent; 111a-proximal end of stent-graft; 111b-distal end of stent-graft; 1111-transverse stent strut; 1112-longitudinal stent strut; 112-coating; 112a-first membrane; 112b-second membrane; 1121-extension part; 120-braided stent; 120a-proximal end of braided stent; 120b-distal end of braided stent; 130-wire-end binding structure; 140-connecting band; 150-proximal radiopaque structure; 160-distal radiopaque structure; I-first structural region; II-second structural region; III-labeled region; 10-liver tissue.

### DETAILED DESCRIPTION

Embodiments of the present invention will be described below by way of specific examples. Based on the disclosure and teachings provided herein, a person of ordinary skill in the art will readily realize other advantages and benefits provided by the present invention. The present invention may also be otherwise embodied or applied through different embodiments, and various modifications or changes may be made to the details disclosed herein from different points of view or for different applications, without departing from the spirit of the present invention. It should be noted that the accompanying drawings are provided herein merely to schematically illustrate the basic concept of the present invention. Accordingly, they only show components relating to the present invention but not necessarily depict all the components as well as their real shapes and dimensions in practical implementations. In practice, the configurations, counts and relative scales of the components may vary arbitrarily and their arrangements may be more complicated.

In the following, each of the embodiments is described as having one or more technical features. However, this does not mean that the present invention must be practiced necessarily with all such technical features, or separately with some or all the technical features in any of the embodiments. In other words, as long as the present invention can be put into practice, a person skilled in the art may choose some or all of the technical features in any of the embodiments or combine some or all of the technical features in different embodiments based on the teachings herein and depending on relevant design specifications or the requirements of practical applications. In this way, the present invention can be carried out more flexibly.

As used herein, the singular forms "a", "an" and "the" include plural referents, and the plural form "plurality" means "two or more", unless the context clearly dictates otherwise. As used herein, the term "or" is generally employed in the sense including "and/or", unless the context clearly dictates otherwise. As used herein, the terms "mounting", "connecting", "coupling" and grammatical variants thereof should be interpreted in a broad sense. For example, a connection may be a permanent, detachable or integral connection, or a mechanical or electrical connection, or a direct or indirect connection with one or more intervening media, or an internal communication or interaction between two components. Those of ordinary skill in the art can understand the specific meanings of the above-mentioned terms herein, depending on their context. As used herein, the terms "proximal end" and "distal end" are employed to describe orientations, positions and directions of components of a stent implant or actions thereon relative to one another, as viewed by a surgeon who is operating the stent implant. "Distal end" is usually used to describe an end that enters a patient's body first, in contrast to "proximal end" being usually used to describe an end closer to the surgeon, although these terms are not intended to be limited to being used in such a way. As used herein, "at least one end" is meant to refer to one end, either proximal end or distal end, or two ends, i.e., both proximal and distal ends.

Objects, features and advantages of the present invention will become more apparent upon reading the following more detailed description thereof in conjunction with the accompanying drawings. Note that the figures are provided in a very simplified form not necessarily drawn to exact scale for the only purpose of helping explain the disclosed embodiments in a more convenient and clearer way.

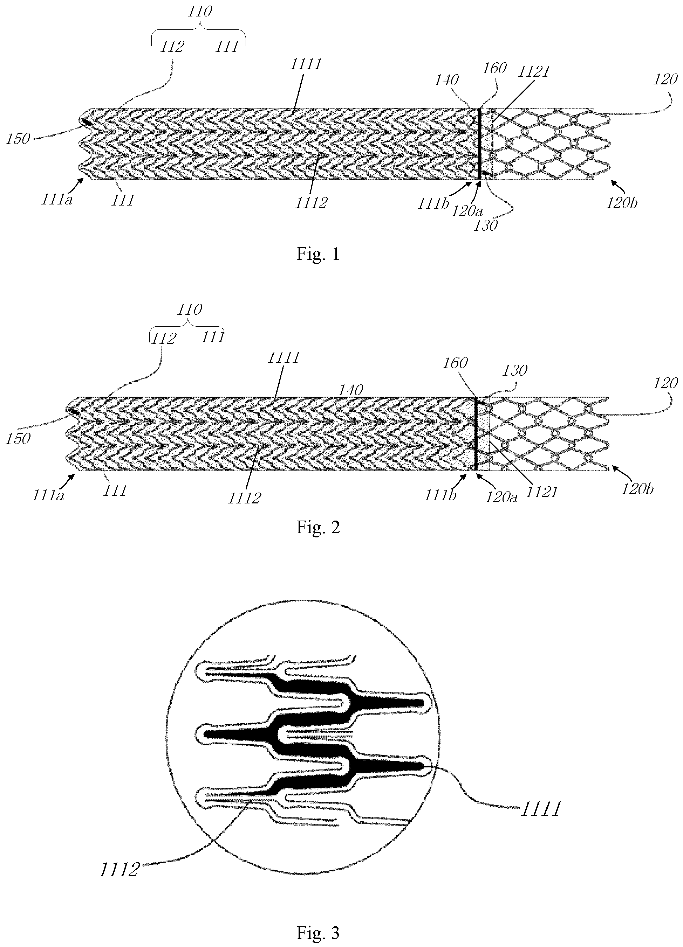

Figs. 1 and 2 is a schematic diagram showing the structure of a stent implant according to a preferred embodiment of the present application. Referring to Figs. 1 to 2, the stent implant includes a stent-graft 110 and a braided stent 120. The stent-graft 110 includes a cutting stent 111 and a coating 112. The coating 112 covers a surface of the cutting stent 111. Typically, the entire surface of the cutting stent 111 is covered by the coating 112. At least one axial end of the stent-graft 110 is connected to the braided stent 120. It will be understood that the cutting stent 111 can be obtained by subjecting a cut tube to heat setting, and the braided stent 120 can be obtained by subjecting a wire-braided tube to heat setting.

The stent implant is configured for implantation at a predetermined site in the body of a patient. For example, in a transjugular intrahepatic portosystemic shunt (TIPS) procedure, it may be implanted within the liver to create a blood flow shunt between the hepatic vein and the portal vein. In this case, the braided stent 120 is configured to allow blood to be supplied to the liver tissue to prevent its atrophy. Approximately 0.5 -1 ring of mesh openings of the braided stent 120 at proximal end may be covered by the coating 112, while the rest of the braided stent 120 may not include the coating 112.

In the stent implant disclosed herein, the cutting stent 111 is combined with the braided stent 120 to provide both good support, which allows a robust blood flow shunt to be created in the liver, and good compliance, which enables the stent implant to be smoothly advanced to the predetermined site. It is noted that the stent implant may include a single braided stent 120 and, in this case, the braided stent 120 may be arranged at a distal end 111b of the stent-graft 110 so that, when the stent implant is implanted in the liver, the braided stent 120 is at the portal vein, while the stent-graft 110 is located within the shunt in the hepatic vein and liver tissue.

Preferably, the stent implant employs a self-expending stent design. The cutting stent 111 consists of stent struts including both transverse stent struts 1111 and longitudinal stent struts 1112. The transverse stent struts 1111 make up annular stent sections, the plurality of annular stent sections are arranged side-by-side along an axis of the cutting stent 111. The longitudinal stent struts 1112 are connected to the annular stent sections, and each annular stent section consists of a plurality of end-to-end joined transverse stent struts 1111 and comprises the shape of waves. The present invention is not limited to any particular shape of such waves. For example, they may be sinusoidal or triangular. Along the circumference of the stent implant, each annular stent section may comprise 6-8 crests, and the trough is provided between adjacent crests. In addition, any two adjacent annular stent sections may be connected by a plurality of longitudinal stent struts 1112, in order to enable effective transmission of pushing forces, which can ensure good delivery of the stent implant. Moreover, this can reduce axial shrinkage of the stent implant, ensuring accurate positioning. The plurality of longitudinal stent struts 1112 that connects adjacent annular stent sections are arranged circumferentially around the cutting stent 111, preferably equidistantly. It will be understood that adjacent annular stent sections are crest-to-crest or trough-to-trough connected by the longitudinal stent struts 1112. In order to ensure good compliance of the cutting stent 111, only some crests or troughs in each annular stent section are connected to the longitudinal stent struts 1112. For example, along the circumference of the stent implant, the longitudinal stent struts 1112 may be spaced at intervals of one or more crests or troughs. For example, each annular stent section may have 6 crests or troughs, and the longitudinal stent struts 112 may be spaced at intervals of one crest or trough. That is, adjacent annular stent sections are connected by three longitudinal stent struts 112.

The braided stent 120 may be braided with wires with a diameter of 0.15 mm to 0.25 mm, preferably 0.20 mm to 0.25 mm. These wires may be made of a shape memory alloy, in particular at least one of Au-Cd, Ag-Cd, Cu-Zn, Cu-Zn-Al, Cu-Zn-Sn, Cu-Zn-Si, Cu-Sn, Cu-Zn-Ga, Au-Cu-Zn, NiAl, Fe-Pt, Ti-Ni, Ti-Ni-Pd, Ti-Nb, U-Nb and Fe-Mn-Si, with Ti-Ni being preferred. The braided stent 120 may have an axial length of 15 mm to 45 mm, preferably 20 mm to 40 mm. The braided stent 120 may have an inner diameter of 6 mm to 12 mm, which is the same as an inner diameter of the cutting stent 111. The braided stent 120 comprises mesh openings, which are typically rhombic. Along an axis of the stent implant, the number of the rhombic mesh openings may range from 3 to 9, preferably 3 to 8, more preferably 4 to 8. Along the circumference of the stent implant, the number of the rhombic mesh openings may range from 6 to 8, which is the same as the number of crests or troughs in each annular stent section of the cutting stent 111.

The stent implant further includes wire-end binding structures 130 disposed on the braided stent 120 to retain ends of the wires to avoid the braided stent 120 from disintegration. In some embodiments, the wire-end binding structures 130 are provided as sleeves with an inner diameter slightly greater than an outer diameter of the wires, allowing end portions of the wires to be inserted and retained in the sleeves. Depending on the diameter of the wires, the inner diameter of the sleeves may be 0.0075 inches to 0.010 inches, preferably 0.0085 inches to 0.0095 inches. The number of wire-end binding structures 130 is the same as the number of wires in the braided stent 120. For example, if the braided stent 120 is braided with only one wire, then one wire-end binding structure is provided. As another example, if the braided stent 120 is braided with two wires, then two wire-end binding structures are provided to retain respective wire end portions. Additionally, the wire-end binding structures 130 may be made of at least one of an elastic metal material and a radiopaque material. For example, they may be made of at least one of a platinum-iridium alloy, a platinum-tungsten alloy, stainless steel, gold, tantalum and a nickel-titanium alloy, preferably a nickel-titanium alloy. In some embodiments, the wire-end binding structures 130 may be sleeves optionally with a length of 2 mm to 4 mm, preferably 2.5 mm to 3.0 mm. Further, the wire-end binding structures 130 may be welded to surfaces of the wires, for example, by laser welding, to securely connect the wire-end binding structures 130 to the wires in the braided stent 120.

For example, the braided stent 120 may be disposed at a distal end of the cutting stent 111. In this case, as shown in Fig. 2, the coating 112 may have an extension part 1121 extending beyond the axial distal end 111b of the cutting stent 111. This extension part 1121 covers a surface of a proximal portion of the braided stent 120 proximate the cutting stent 111 (e.g., it may cover 0.5-1 ring of mesh openings at a proximal end of the braided stent 120 along an axial direction) and directly thermally fused to the braided stent 120. Additionally or alternatively, as shown in Fig. 1, the stent implant may further include connecting bands 140, which are passed through mesh openings of the braided stent 120 around an interface of the braided stent 120 and the cutting stent 111 and thermally fused to the coating 112. Additionally, the connecting bands 140 are attached to some mesh openings of the braided stent 120 that are proximate a proximal end of the cutting stent 111. In this way, the connection between the connecting band 140 and the braided stent 120 is equivalent to a hinge connection, making the braided stent 120 more flexible. The connecting bands 140 may be made of the same material as the coating 112, or not. Preferably, the connecting bands 140 are made of the same material as the coating 112. For example, the connecting bands 140 may be made of the same material as first membrane.

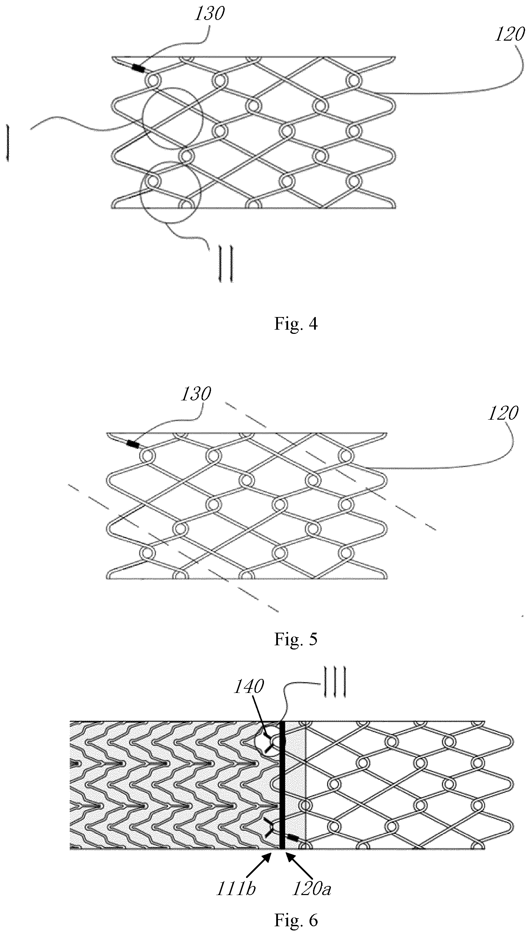

As shown in Fig. 6, a connecting band 140 may be provided in a region of the stent implant, labeled as III, where the proximal end 120a of the braided stent 120 is connected to the distal end 111b of the stent-graft 110. The connecting bands 140 are preferably made of polytetrafluoroethylene (PTFE). In the example with the braided stent 120 being disposed at the distal end of the cutting stent 111, each connecting band 140 may be passed through a corresponding mesh opening in the braided stent 120 (in a region III) around the interface of the braided stent 120 and the cutting stent 111, and its two ends may be then arranged on the cutting stent 111 and thermally fused to the coating 112. This enables more secure attachment of the connecting bands 140 to the coating 112. Moreover, since connecting bands 140 comprises a certain length along a axial direction, that is, the connecting bands 140 axially extend a certain length along an axis of the stent-graft 110, this can reduce axial shrinkage of the stent-graft 110, ensuring accurate positioning of the stent-graft 110. It will be understood that, herein, by "a corresponding mesh opening", it is intended to mean that each connecting band 140 is passed through only one selected mesh opening, and different connecting bands 140 are passed through different mesh openings.

However, it is undesirable for the connecting bands 140 to extend an excessive length on the stent-graft 110, because this would reduce compliance of the stent-graft 110. For this reason, the length of the connecting bands 140 is preferred to be 20 mm to 40 mm. For example, each connecting band 140 may extend 10-20 mm from the distal end 111b of the cutting stent 111 to the joint with the braided stent 120 and then extend 10 mm to 20 mm in the opposite direction. In this way, the connecting bands 140 hook onto the mesh openings of the braided stent 120. The connecting bands 140 not only impart greater compliance to the braided stent 120, but also effectively and more strongly connect the braided stent 120 and the cutting stent 111 to each other, which are separately fabricated using different techniques.

Referring back to Figs. 1 to 2, at least one proximal radiopaque structure 150 may be provided on the stent-graft 110 at an axial proximal end 111a thereof. Radiopacity of the proximal radiopaque structure 150 to X-rays enables locating of the proximal end of the stent-graft 110. The proximal radiopaque structure 150 is provided typically as a radiopaque sleeve disposed over a transverse stent strut 1111. At least one distal radiopaque structure 160 may also be provided on the stent-graft 110 at the distal end 111b thereof. Radiopacity of the distal radiopaque structure 160 to X-rays enables locating of the distal end of the stent-graft 110. Preferably, the distal radiopaque structure 160 is provided as a radiopaque marker band disposed at the interface of the cutting stent 111 and the braided stent 120. In case of the stent implant including a single braided stent 120, the distal radiopaque marker band may be arranged closer to the braided stent 120. The present invention is not limited to any particular material from which the proximal radiopaque structure 150 and/or the distal radiopaque structure 160 is/are fabricated. Examples of suitable materials include platinum-iridium alloys, platinum-tungsten alloys, stainless steel, gold and tantalum. Among them, tantalum and gold are preferred, and gold is more preferred.

The coating 112 is made of a polymer material for medical use, which should possess good ductility and permeability. Optionally, it may be polyethylene terephthalate (PET), or expanded PTFE (ePTFE), or a combination of ePTFE and fluorinated ethylene propylene (FEP).

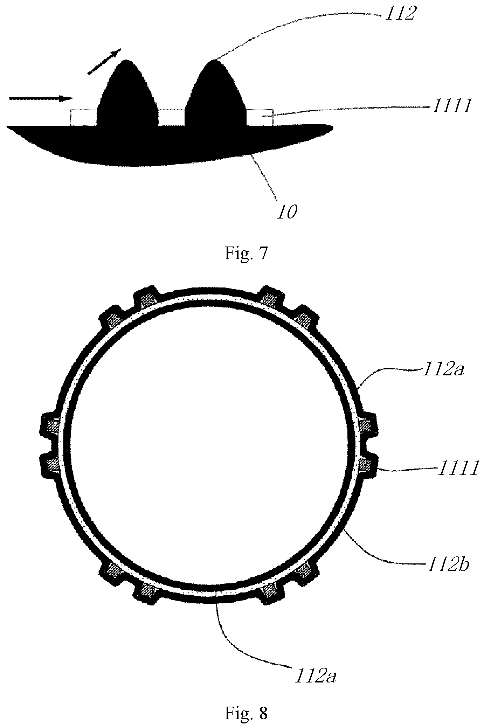

Referring to Fig. 8, in an embodiment of the present application, the coating 112 includes two first membranes: an outer first membrane 112a covering an outer surface of the cutting stent 111; and an inner first membrane 112a covering an inner surface of the cutting stent 111. The outer first membrane 112a exhibits lower air permeability than the inner first membrane 112a. For each first membrane 112a, the air permeability depends on its pore size. A larger size means higher air permeability and a shorter air permeability time; and vice versa. Herein, an air permeability time is defined as a period of time required for a given volume of air to pass through a membrane. With this arrangement, the stent implant of the present invention can provide good support and flexibility. For example, when used in a TIPS procedure, two first membranes are arranged, the less air-permeable outer first membrane 112a disallows the permeation of bile due to a denser texture, and the two first membranes 112a with different degrees of air permeability impart increased compliance to the stent-graft 110. Specifically, the inner first membrane material 112a which has a higher air permeability comprises larger pore sizes and better flexibility. In contrast, the outer first membrane material 112a with lower air permeability is less flexible than the inner first membrane material 112a with higher air permeability. By increasing the flexibility of the highly permeable inner first membrane material 112a, the overall flexibility of the stent-graft 110 is improved. At the same time, the influence of the thermal fusing processes on the compliance of the stent-graft 110 is mitigated. It will be understood that, since the more air-permeable first membrane 112a has larger pores, it is more compliant, increasing overall compliance of the stent-graft 110. Thus, in addition to providing better support and maintaining a blood flow shunt, the stent-graft 110 can adapt itself to the geometry of the blood flow shunt and reduce damage to a blood vessel. Further, the outer and inner first membranes 112a with different degrees of air permeability can be fused and joined to each other with higher strength, enhancing the bonding force between the outer first membrane 112a and inner first membrane 112a, thereby preventing delamination, reducing the risk of a lumen of the stent-graft 110 becoming stenotic or occluded. For example, during thermal fusion of the coating 112, the different pore sizes of the two first membranes 112a allow the molten material of each membrane to desirably more penetrate into the other membrane, with the increased penetration, a stronger adhesion force between the two first membranes 112a can be achieved.

The first membranes 112a are preferably made of an ePTFE material because such ePTFE membranes exhibit good ductility and good permeability. For example, an air permeability time of the outer first membrane may be 6.0 s to 20.0 s, and an air permeability time of the inner first membrane may be 0.5 s to 6.0 s. For each first membrane 112a, a greater thickness means a harder texture and hence lower compliance. Accordingly, in this embodiment, each first membrane 112a has a thickness of 20 µm to 180 µm, preferably 20 µm to 120 µm, more preferably 20 µm to 60 µm.

Preferably, the coating 112 further includes second membrane(s) 112b with a glass transition temperature lower than a glass transition temperature of the first membrane 112a. The second membrane 112b is disposed between the outer first membrane 112a and the inner first membrane 112a at the proximal end (i.e., one end) of the cutting stent 111. Additionally or alternatively, the second membrane 112b is provided between the outer first membrane 112a and the inner first membrane 112a at the distal end (i.e. the other end) of the cutting stent 111. It will be appreciated that no second membrane 112b is provided over an intermediate section of the cutting stent 111 between its distal and proximal ends. The second membranes 112 are provided to enhance adhesion force of the outer first membrane 112a and the inner first membrane 112a at the two ends of the cutting stent 111, avoiding delamination (separation) of the two first membranes 112a at the ends under the impact of blood flow. This can additionally reduce the risk of the lumen of the stent implant becoming stenotic or occluded.

Additionally, the second membrane 112b may be provided between the outer surface of the cutting stent 111 and the outer first membrane 112a, or between the inner surface of the cutting stent 111 and the inner first membrane 112a, at the axial proximal end of the cutting stent 111. The, second membrane 112b may be provided between the outer surface of the cutting stent 111 and the outer first membrane 112a, or between the inner surface of the cutting stent 111 and the inner first membrane 112a, at the axial distal end of the cutting stent 111. The second membrane 112a is preferably made of an FEP material and it has a glass transition temperature (about 220 °C) lower than that (about 320 °C) of the ePTFE material. In this way, the FEP membrane(s) can be molten to effectively enhance adhesion force of the two ePTFE membranes at either or both ends of the cutting stent 111.

Each second membrane 112a may have a thickness of 10 µm to 30 µm, preferably 10 µm to 15µm, and may be cut into a strip wrapped around a surface (e.g., the outer or inner surface) of the cutting stent 111. In case of the second membrane being selected as an FEP material, which is harder than the ePTFE material, its width along the axis of the stent-graft 110 is not desired to be too large. Otherwise, the compliance of the stent-graft 110 will be reduced. Preferably, the width of the second membrane 112a at the proximal or distal end along the axis of the stent-graft 110 accounts for 3% to 18% of a total axial length of the stent-graft 110. It should be noted that, when the second membrane 112b is provided on the inner or outer surface of the cutting stent 111 at its axial distal end, the coating 112 typically has the extension part 1121 extending beyond the distal end of the cutting stent 111. In this case, the second membrane 112b may also have an extension part 1121 provided between an extension part 1121 of the outer first membrane 112a and an extension part 1121 of the inner first membrane 112a. Additionally, the extension part 1121 of the second membrane 112b may wrap around a surface (e.g., an inner or outer surface) of the proximal end of the braided stent 120 proximate the cutting stent 111. When the extension part 1121 of the second membrane 112b wraps around an inner surface of the corresponding proximal end of the braided stent 120, it is situated between the inner surface of the corresponding proximal end of the braided stent 120 and the extension part 1120 of the inner first membrane 112a, and the extension part 1121 of the outer first membrane 112a is located on the outer surface of the corresponding proximal end of the braided stent 120. When the extension part 1121 of the second membrane 112b wraps around an outer surface of the corresponding proximal end of the braided stent 120, it is situated between the outer surface of the corresponding proximal end of the braided stent 120 and the extension part 1121 of the outer first membrane 112a, and the extension part 1121 of the inner first membrane 112a is located on the inner surface of the corresponding proximal end of the braided stent 120. Further, the outer first membrane 112a and the inner first membrane 112a are aligned at both ends along the axis of the stent-graft 110. Furthermore, the second membrane(s) 112b (e.g., FEP membrane(s)) also allow(s) fusion of the first membranes 112a (e.g., ePTFE membranes) to the connecting bands 140 (e.g., PTFE bands) and effective connection of the cutting stent 111 and the braided stent 120, which are separately fabricated using different techniques. It will be understood that, as the ePTFE membranes and the PTFE bands contain the same component, in a thermal treatment of the stent implant, after the temperature exceeds the glass transition temperature of FEP (approximately 220 °C), the molten FEP can serve as an intermediary to join the ePTFE membranes (with a glass transition temperature of approximately 320 °C) to the PTFE bands.

Fig. 7 schematically illustrates a comparative example, in which a conventional stent-graft is implanted into the liver in a TIPS procedure so that liver tissue 10 adheres to an outer surface of the stent-graft. However, the postoperative healing and growth of the liver tissue 10 will try to repair the blood flow shunt created in the procedure, exerting compression on the stent-graft. In non-metallic regions (i.e., non-stented regions) of the stent-graft lacking support from a braided stent, the liver tissue 10 grows faster toward the lumen of the stent-graft and squeezes the coating 112. Consequently, the coating 112 may bulge toward the lumen of the stent-graft, leading to an uneven luminal surface of the stent-graft. This reduces the effective cross-sectional area of the lumen for blood flow and slows down blood flow within the lumen (in a direction as indicated by the arrow in the figure). For this reason, thrombosis may occur, which will additionally narrow or even occlude the lumen. This problem is attributable to low surface metal coverage of the braided stent, which makes it fail to provide sufficient support.

In order to circumvent the problem of possible restenosis or occlusion that may arise from the use of conventional braided stents due to low surface metal coverage and hence insufficient support thereof, according to embodiments of the present application, metal coverage of the cutting stent 111 may be as high as 10% to 20%, more preferably 11% to 17%. The metal coverage not only enables the stent-graft 110 to provide increased radial support, but also takes into account its compliance. Therefore, according to embodiments of the present application, the stent-graft 110 can well resist squeezing of liver tissue 10 and prevent an uneven surface of the lumen of the stent-graft 110, ensuring that the lumen's effective cross-sectional area for blood flow will not be reduced. Accordingly, blood flow within the lumen of the stent-graft will not be affected, reducing the risks of thrombosis and of restenosis or occlusion of the stent-graft when used in a TIPS produce. It will be understood that surface metal coverage of 10% to 20%, or 11% to 17%, means shorter distances between adjacent stent struts, which can greatly reduce and uniformize bulging of the coating 112 when squeezed by the liver tissue 10, reducing its impact on hemodynamics and lowering the risk of thrombosis. It will also be understood that the metal coverage is defined as the percentage of an area of the cutting stent's metal material to total outer surface area of the cutting stent.

In some embodiments, the cutting stent 111 is made of a nickel-titanium alloy, or another shape memory alloy. The cutting stent 111 may have an axial length of 40 mm to 100 mm, preferably 40 mm to 80 mm, more preferably 50 mm to 80 mm. The stent struts 1111 in the cutting stent 111 may have a width of 0.09 mm to 0.25 mm, more preferably 0.09 mm to 0.18 mm. The cutting stent 111 may have a wall thickness of 0.15 mm to 0.25 mm, preferably 0.18 mm to 0.24 mm. The cutting stent 111 may have an inner diameter of 6 mm to 12 mm.

The stent struts in the cutting stent 111 (including both the transverse stent struts 1111 and the longitudinal stent struts 1112) are preferably surface-coated with a connecting layer for enhancing adhesion force of the coating 112 to the stent struts. The connecting layer may be made of the same material as the coating 112, or not. For example, when the first membranes 112a are made of an ePTFE material, the connecting layer may be made of an ePTFE or FEP material. In particular embodiments of the present application, the connecting layer may be formed by spraying of, or dipping in, an ePTFE or FEP solution. For example, in the latter case, the entire cutting stent 111 may be separately dipped in a solution for forming the connecting layer. After that, the cutting stent 111 may be taken out and subjected to a curing process, forming the connecting layer that coats the surface of the stent struts. In order to ensure uniform coverage of the connecting layer, the curing process may be followed by repeated dipping cycles, for example, 2-5 repeated dipping cycles, preferably 2-3 repeated dipping cycles. It will be understood that the connecting layer is not present at the gaps between adjacent stent struts. That is, the connecting layer is only present on the stent struts. The connecting layer may serve as an intermediate layer, which is attached to the stent struts while being thermally fused to the coating 112. This can enhance adhesion force of the coating 112 on the surface of the cutting stent 111 to the stent struts and reduce the probability of separation and delamination of the coating 112 from the stent struts, thereby additionally lowering the risk of the lumen of the stent-graft becoming stenotic or occluded. The solution for forming the connecting layer is preferred to be an ePTFE or FEP solution. In the solution, ePTFE or FEP may be present at 2% to 10%, preferably 2% to 5% by weight. The curing process may involve natural drying, or drying in an oven.

Referring to Fig. 4, the braided stent 120 may be braided with at least one wire, and may be provided with first structural regions I and second structural regions II. In the first structural regions I, different wire portions simply overlap, with each wire portion running above or below another wire portion. In the second structural regions II, different wire portions are in inseparable engagement, with each wire portion looped around another wire portion. The first structural regions I impart anisotropic compliance to the braided stent 120, and the second structural regions II ensure sufficient compliance of the braided stent 120. However, if all the first structural regions I are aligned along one line extending parallel to an axis of the braided stent 120, bending of the braided stent 120 would be restricted. That is, the braided stent 120 is not bendable at will. This will indirectly lead to a decrease in compliance. In order to avoid this, preferably, all the first structural regions I are arranged along the axis of the braided stent 120 into a helical pattern, which makes the braided stent 120 freely 360° bendable. That is, its bending is not restricted in any direction. With this arrangement, the braided stent 120 can better accommodate the geometry of a blood vessel.

Use of the stent implant in a TIPS procedure will be described below, as an example.

At first, an operator completes all preparatory operations necessary for the shunt procedure and preoperative assessment. After that, the operator calculates a portal pressure gradient (PPG) value based on the preoperative assessment and determines suitable diameters (inner and outer diameters) and a suitable length for the stent implant. A stent system (including the stent implant and a delivery device for delivering the stent implant) is then delivered over a guidewire within a sheath of a puncture catheter into the liver. Subsequently, the sheath is withdrawn, and the stent system is manipulated to release the braided stent 120 and a portion of the cutting stent 111 and then the entire stent system is retracted. The stent system is then retracted until the distal radiopaque structure 160 is located at the distal end of the portal vein (PV)/parenchyma junction. Afterwards, the stent system is manipulated to release the entire cutting stent 111, followed by withdrawal of the delivery device. If required, a balloon may be then introduced and used to act on the stent implant to additionally expand the cutting stent to a desired inner diameter. Thereafter, with the aid of portography, the location and lumen opening of the implant are recorded. If any folding, twisting, collapse or incomplete expansion of the stent is found, the operator may then again introduce the balloon to re-expand the stent implant to achieve a larger shunt.

Embodiments of the present invention also provide a method for preparing the stent implant according to any of the foregoing embodiments. The method includes the steps of:
disposing the inner first membrane 112a over a mold;
sequentially disposing the cutting stent 111 and the braided stent 120 over the inner first membrane 112a so that an entire inner surface of the cutting stent 111 and an inner surface of a portion of the braided stent 120 proximate one end (e.g., the proximal or distal end) of the cutting stent 111 are covered by the inner first membrane 112a;
disposing the outer first membrane 112a over the cutting stent 111 and the outer first membrane 112a covers an entire outer surface of the cutting stent 111 and an outer surface of a portion of the braided stent 120 proximate said one end (e.g., the proximal or distal end) of the cutting stent 111; and
covering the outer first membrane 112a by a heat shrink tube, then carrying out a heating and cooling cycle, and finally removing the mold and the heat shrink tube.

In one embodiment, the method further includes:
after the inner first membrane 112a is placed on the mold and before the cutting stent 111 and the braided stent 120 are disposed over the inner first membrane 112a, disposing second membrane(s) 112b between the inner first membrane 112a and the inner surface of the cutting stent 111 at at least one axial end of the cutting stent 111; and
after the cutting stent 111 and the braided stent 120 are disposed, disposing the distal radiopaque marker band at the interface of the cutting stent 111 and the braided stent 120 and, after the distal radiopaque marker band is disposed, disposing the outer first membrane 112a over the cutting stent 111.

In one preferred embodiment, the method further includes:
before the outer first membrane 112a is disposed over the cutting stent 111, disposing second membrane(s) 112b between the outer surface of the cutting stent 111 and the outer first membrane 112a at at least one axial end of the cutting stent 111; and
after the second membrane(s) 112b is/are disposed, disposing the distal radiopaque marker band at the interface of the cutting stent 111 and the braided stent 120 at a certain distance from a corresponding end (e.g., the distal end) of the inner first membrane 112a and, after the distal radiopaque marker band is disposed, disposing the outer first membrane 112a over the cutting stent 111.

In one embodiment, before the outer first membrane 112a is disposed over the cutting stent 111, the method further includes: disposing the connecting bands 140 at one axial end (e.g., the distal end) of the cutting stent 111, and the connecting bands 140 are each passed through a corresponding mesh opening in the braided stent 120 at the interface of the braided stent 120 and the cutting stent 111 and thus engage some mesh openings in the braided stent 120 proximate said one end of the cutting stent 111.

Preferably, the method further includes: wrapping the heat shrink tube with a film; and after the film is applied, carrying out a thermal treatment process.

It will be understood that providing the film wrapped over the heat shrink tube can additionally enhance adhesion force of the coating 112 to the cutting stent 111.

A more detailed example is set forth below, in which ePTFE membranes are used as the first membranes 112a, FEP membranes are used as the second membranes 112b and PTFE bands are used as the connecting bands 140 and a single braided stent 120 is arranged at the distal end of the cutting stent 111.

In a non-limiting embodiment, the method of preparing the stent implant may include the steps as follows:
Step 1: Wrap a more air-permeable ePTFE membranes over an appropriately-sized mold as an inner ePTFE membrane. In this step, the inner ePTFE membrane wrapped over the mold may be provided as a sheet or tube.
Step 2: Sequentially dispose the cutting stent 111 and the braided stent 120 over the inner ePTFE membrane in the order as shown in Fig. 1, adjust the length of the inner ePTFE membrane so that the inner ePTFE membrane cover the entire inner surface of the cutting stent 111 and 0.5-1 ring of mesh openings in the braided stent 120 proximate the proximal end of the cutting stent 111, and connect the cutting stent 111 to the braided stent 120 through multiple PTFE bands (i.e., the connecting bands 140) by a loop back manner.
Step 3: Dispose the FEP strips over the outer surface of the cutting stent 111 respectively at the axial proximal and distal ends as the second membranes 112b, and wrap the distal radiopaque marker band at the interface of the cutting stent 111 and the braided stent 120, the distal radiopaque marker band is spaced at 2 mm to 3 mm from the most distal position of the inner ePTFE membrane.
Step 4: Wrap the other less air-permeable ePTFE membrane over the outer surface of the cutting stent 111 as an outer ePTFE membrane, the outer ePTFE membrane is aligned with the inner ePTFE membrane at both ends. In this step, the outer ePTFE membrane may be provided as a sheet or tube.
Step 5: After these preparatory operations are completed, cover the outer ePTFE membrane (i.e., the resulting coating) by a heat shrink tube with an appropriate inner diameter.
Step 6: Tightly warp the heat shrink tube with a further ePTFE membrane (i.e., the aforementioned film).
Step 7: Subject the resulting structure to thermal treatment in an oven at a temperature higher than the glass transition temperature of the ePTFE membranes for 5-10 minutes.
Step 8: After the thermal treatment is completed, remove the heat shrink tube and the wrapping ePTFE membrane after they are cooled and cured. Finally, the resulting stent implant is removed from the mold.

It is noted that many modifications and additions may be made by those of ordinary skill in the art without departing from the teachings disclosed hereinabove, and such modifications and additions are intended to be included within the scope of the present invention. Any and all equivalent changes such as alternations, modifications and variations made in light of the above disclosure by those familiar with the art without departing from the spirit and scope of the invention are all considered as equivalent embodiments of the present invention. Accordingly, any and all equivalent changes such as alternations, modifications and variations made to the disclosed embodiments in accordance with the essential principles of the present invention fall within the scope of the invention.

## Claims

1. A stent implant, comprising a stent-graft and a braided stent, wherein at least one axial end of the stent-graft is connected to the braided stent, wherein the stent-graft comprises a cutting stent and a coating, wherein the coating comprises two first membranes , wherein the outer first membrane is disposed over an outer surface of the cutting stent, wherein the inner first membrane is disposed over an inner surface of the cutting stent, and wherein the outer first membrane comprises a lower air permeability than the inner first membrane.

2. The stent implant according to claim 1, wherein a metal coverage of the cutting stent ranges from 10% to 20%.

3. The stent implant according to claim 1, wherein surfaces of stent struts in the cutting stent are covered with a connecting layer, and wherein the connecting layer is configured to enhance an adhesion force of the coating to the stent struts.

4. The stent implant according to claim 1, wherein the coating further comprises at least one second membrane, wherein the second membrane has a lower glass transition temperature than the first membrane, wherein the second membrane is disposed between the outer and inner first membranes at a proximal end of the cutting stent, and/or the second membrane is disposed between the outer and inner first membranes at a distal end of the cutting stent, and wherein the second membrane is configured to enhance an adhesion force between the outer and inner first membranes.

5. The stent implant according to claim 4, wherein the first membrane is made of an expanded polytetrafluoroethylene (ePTFE) material, and wherein the second membrane is made of a fluorinated ethylene propylene (FEP) material.

6. The stent implant according to claim 4 or 5, wherein a width of the second membrane disposed at the proximal or distal end of the cutting stent along an axis of the stent-graft accounts for 3% to 18% of a total axial length of the stent-graft.

7. The stent implant according to claim 4 or 5, wherein an air permeability time of the outer first membrane is 6.0 s to 20.0 s, wherein an air permeability time of the inner first membrane is 0.5 s to 6.0 s, wherein the first membrane has a thickness of 20 µm to 180 µm, and wherein the second membrane has a thickness of 10 µm to 30 µm.

8. The stent implant according to claim 4 or 5, wherein the coating has an extension part extending beyond one of the axial ends of the cutting stent, wherein an extension part of the second membrane is disposed between an extension part of the outer first membrane and an extension part of the inner first membrane, and wherein the extension part of the second membrane covers a surface of a portion of the braided stent proximate a corresponding end of the cutting stent.

9. The stent implant according to any one of claims 1 to 5, wherein the coating is thermally fused to the braided stent, and/or wherein the stent implant further comprises a plurality of connecting bands, wherein each connecting band is passed through a corresponding mesh opening in the braided stent at an interface of the braided stent and the cutting stent and is thermally fused to the coating.

10. The stent implant according to claim 3, wherein the connecting layer is made of an FEP or ePTFE material.

11. The stent implant according to claim 10, wherein the connecting layer is formed on surfaces of stent struts by dipping an entire cutting stent in an ePTFE or FEP solution, or by spraying an ePTFE or FEP solution on the entire cutting stent.

12. The stent implant according to any one of claims 1 to 5, wherein the braided stent is braided with at least one wire, wherein the braided stent comprises first structural regions and second structural regions, wherein wire portions overlap in the first structural region, wherein wire portions are in mutual engagement in the second structural region, and wherein the first structural regions are arranged into a helical pattern along an axis of the braided stent.

13. The stent implant according to any one of claims 1 to 5, wherein the stent struts include transverse stent struts and longitudinal stent struts, wherein the transverse stent struts form a plurality of annular stent sections arranged side by side along an axis of the cutting stent, wherein the longitudinal stent struts connect the annular stent sections, wherein any adjacent annular stent sections are connected by a plurality of longitudinal stent struts, and wherein only some crests or troughs in each annular stent section are connected to the longitudinal stent struts.

14. A method of preparing the stent implant according to any one of claims 1 to 13, comprising:
disposing an inner first membrane over a mold;
sequentially disposing a cutting stent and a braided stent over the inner first membrane so that an entire inner surface of the cutting stent and an inner surface of a portion of the braided stent proximate one end of the cutting stent is covered by the inner first membrane;
disposing an outer first membrane over the cutting stent, wherein the outer first membrane covers an entire outer surface of the cutting stent and an outer surface of a portion of the braided stent proximate one end of the cutting stent; and
covering the outer first membrane by a heat shrink tube, then performing a heating and cooling cycle and finally removing the mold and the heat shrink tube.

15. The method according to claim 14, wherein after the inner first membrane is disposed over the mold and before the cutting stent and the braided stent are disposed over the inner first membrane, a second membrane having a lower glass transition temperature than the first membranes is disposed between the inner first membrane and the inner surface of the cutting stent at at least one axial end of the cutting stent to enhance an adhesion force between the outer and inner first membranes, and
wherein after the cutting stent and the braided stent are disposed, a distal radiopaque marker band is wrapped around an interface of the cutting stent and the braided stent, wherein the outer first membrane is disposed over the cutting stent after disposal of the distal radiopaque marker band.

16. The method according to claim 14, further comprising, after the cutting stent and the braided stent are disposed over the inner first membrane and before the outer first membrane is disposed over the cutting stent:
disposing a second membrane having a lower glass transition temperature than the first membranes between the outer surface of the cutting stent and the outer first membrane at at least one axial end of the cutting stent to enhance an adhesion force between the outer and inner first membranes; and
after disposal of the second membrane, wrapping a distal radiopaque marker band around an interface of the cutting stent and the braided stent, wherein the outer first membrane is disposed over the cutting stent after disposal of the distal radiopaque marker band.

17. The method according to claim 14, further comprising, before the outer first membrane is disposed over the cutting stent: disposing a plurality of connecting bands at one axial end of the cutting stent, wherein each connecting band is passed through a corresponding mesh opening in the braided stent at an interface of the braided stent and the cutting stent, thereby engaging some mesh openings of the braided stent that are proximate the one end of the cutting stent.

18. The method according to any one of claims 14 to 17, further comprising: wrapping the heat shrink tube with a film; and then performing a thermal treatment process after the film is applied.
